# EUROPEAN PATENT SPECIFICATION

(11) **EP 0 555 324 B1**
(45) Date of publication and mention of the grant of the patent: **12.03.1997**
(21) Application number: 91919728.5
(22) Date of filing: 31.10.1991
(51) Int. Cl.: C07C 227/40, C07C 227/28

(54) **Process for separating basic, acidic and amphoteric amino acids by electrodialysis**
Elektrodialytisches Verfahren zur Trennung von basischen, sauren und amphoteren Aminosäuren
Separation d'acides aminés acides, basiques et amphotères par électrodialyse

(30) Priority: 02.11.1990 FR 9013610
(43) Date of publication of application: 18.08.1993
(73) Proprietor: SMITHKLINE BEECHAM LABORATOIRES PHARMACEUTIQUES, F-92731 Nanterre Cédex (FR)
(72) Inventor: LE GOFF, Jean-Pierre, F-53101 Mayenne Cédex (FR); GAVACH, Claude, CNRS, Lab. de Physico-Chimie des, F-34033 Montpellier Cédex (FR); SANDEAUX, Roger, CNRS, Lab. de Physico-Chimie des, F-34033 Montpellier Cédex (FR); SANDEAUX, Jacqueline, CNRS, Lab. de Physico-Chimie, F-34033 Montpellier Cèdex (FR)
(74) Representative: Rutter, Keith, Dr.
(86) International application number: EP9102077
(87) International publication number: WO9207818

(56) References cited:
- US-A- 3 051 640
- US-A- 3 231 485
- US-A- 3 330 749

## Description

This invention relates to a process for separating a mixture of amino acids into its component species (basic, acid and amphoteric) and in particular a separation process using electrodialysis.

Amino acids are frequently extracted from hydrolysates of animal and vegetable proteins. On an industrial scale, protein wastes from animal food processing are subjected to hydrolysis to produce a complex mixture of amino acids, and this mixture can be separated by, for example, fractional precipitation and ion exchange techniques into individual amino acids. However, these processes are expensive, require the consumption of large amounts of chemical reagents and produce considerable quantities of potentially polluting effluents.

A process has now been devised which permits the separation of an amino acid mixture into its component species by means of a multi-compartment electrodialysing apparatus, thereby reducing the quantities of chemical reagents required and the effluent problem.

According to the present invention there is provided a method of separating the acidic, basic and amphoteric components of an aqueous mixture of amino acids, which comprises introducing the mixture into a first electrodialyser chamber bounded by a cation exchange membrane and an anion exchange membrane, applying an electric potential across the chamber to cause the basic components to pass through the cation exchange membrane into a second electrodialyser chamber bounded by a further anion exchange membrane, and the acidic components to pass through the anion exchange membrane into a third electrodialyser chamber bounded by a further cationic exchange membrane, and maintaining the electric potential to permit the basic components and acidic components to accumulate in the second and third chambers respectively, thereby leaving the amphoteric components in the first chamber; characterised in that all chambers are subsequently drained and the various membranes are subjected to a rinse cycle in order to minimise fouling or clogging.

Preferably, the amino acid mixture comprises a protein hydrolysate, and therefore has a high content of acidic amino acids, especially aspartic acid and glutamic acid, basic amino acids, especially lysine and arginine, and amphoteric acids, especially glycine, serine, proline and hydroxyproline.

Preferably, the second and third chambers contain an aqueous solution of a sodium salt, particularly sodium chloride, as the conducting medium, and a fourth electrodialysing chamber also containing a sodium salt is located adjacent to the second chamber, so as to create a set of four chambers in parallel.

Preferably, a plurality of sets of chambers are provided, wherein all the first chambers are connected to each other in series, and each second, third and optionally fourth chamber is also connected to the other second, third and optionally fourth chamber respectively.

This arrangement permits liquid in each first chamber to circulate freely throughout the remaining first chambers, and likewise for the second, third and fourth chambers. Suitably, at least four sets of chambers are provided, although the number may be increased according to the volume of amino acid mixture to be separated into components.

Usually, the aqueous mixture of amino acids introduced into the first chamber will already contain a certain amount of salt, such as sodium chloride, and preferably the identical salt is also present in aqueous solution in the second and third chambers at the beginning of the electrodialysis process. During the process, the salt cations in the first chamber pass into the second chamber, while the salt anions pass into the third chamber. The result of this is that virtually all salt present in the aqueous mixture in the first chamber is removed therefrom.

Suitably, the cation and anion exchange membranes bounding the first chamber are of the 'loose' type, and examples are those manufactured by Morgane, type CDS 160% for the cation membrane, and type ADP for the anion membrane.

The 'loose' type of membrane allows the passage of large and small ions, so that both amino acid and salt ions can pass through.

The anionic membrane bounding the second chamber, and the cationic membrane bounding the third chamber, are preferably of the 'dense' type and examples are Morgane type CDS for the cation membrane, and type ADS for the anion membrane. The 'dense' type of membrane allows the passage of small ions only, so that the salt ions can pass through but not the amino acid ions.

Both the 'loose' and 'dense' type membranes are characterised by having a good resistance to hydroxyl ions, provide a strong barrier to the transport of water, and have a low tendency to fouling or clogging as electrodialysis proceeds.

It has been found that an initial concentration of about 10⁻²M of salt in the second and third chambers is required to obtain the optimum current density for the electrodialysis process, and the preferred current density is from 20mAcm⁻² to 40mAcm⁻², more preferably about 30mAcm⁻².

The concentration of salt in the fourth chamber is usually greater than that in the second or third chambers, and an initial concentration of about 0.5M has been found to be particularly suitable.

To commence the process of the invention, the aqueous mixture is pumped into each first chamber, and the appropriate salts into the remaining chambers. Current is applied across the chambers, and as a result each second chamber becomes enriched in basic amino acids, and each third chamber enriched in acidic amino acids. When virtually all basic and acidic amino acids have been removed from each first chamber, leaving just the amphoteric amino acids, all chambers are drained and the various membranes are subjected to a rinse cycle in order to minimise fouling or clogging. After rinsing, further amino acid mixture and salts are pumped into the appropriate chambers and the process is repeated. The rinse cycle of the invention comprises washing the membranes in a sequence consisting of water, dilute hydrochloric acid, dilute sodium chloride, dilute sodium hydroxide and finally dilute sodium chloride.

The basic amino acid solution drained from each second chamber will also be rich in salt, and separation of the salt can be carried out using a conventional demineralisation electrodialysis method with a 2-compartment electrodialyser. Similarly the acidic amino acid solution drained from the third chambers will also be rich in salt, and separation of the salt can occur as for the basic amino acids. Each of the basic, acid and amphoteric components can then be treated by standard chemical separation techniques, such as fractional crystallisation, recrystallisation or ion exchange methods, to obtain individual amino acids.

When the original amino acid mixture is in the form of a protein hydrolysate, this may be prepared by first decolourising a crude hydrolysate medium, produced from hydrolysing protein wastes, and demineralising the decolourised hydrolysate using electrodialysis.

The decolourisation is preferably performed by treating the hydrolysate with activated charcoal and regenerating the charcoal with a microfiltration system, in accordance with known techniques. The decolourised hydrolysate may be demineralised using a conventional two compartment electrodialyser, preferably employing compact, dense ion. exchange membranes such as those manufactured by Morgane.

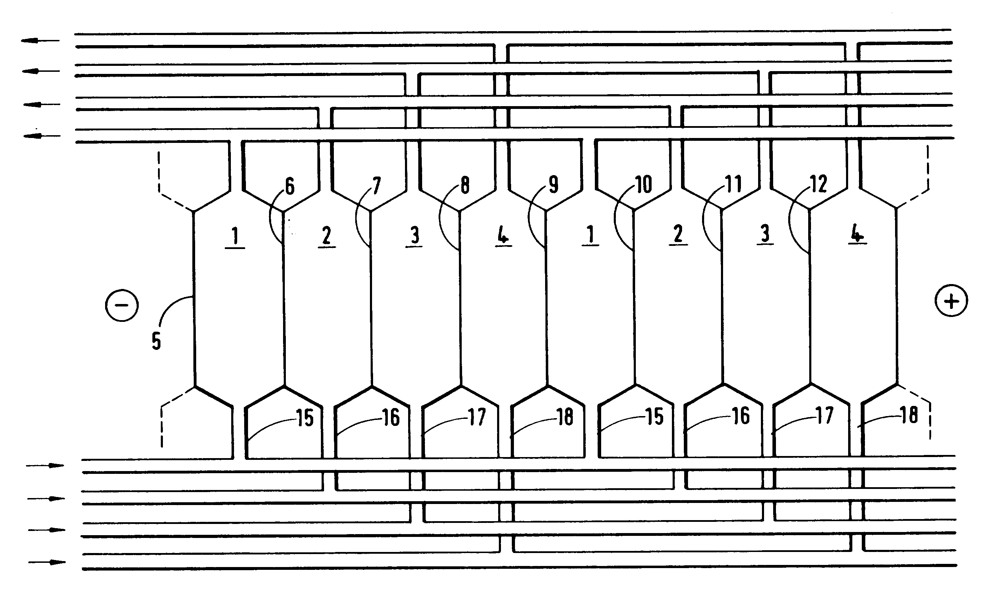

The invention is now described in more detail with reference to the accompanying drawing, which is a schematic view of an electrodialyser for separating acidic, basic and amphoteric amino acids.

Referring to the drawing, an electrodialyser comprises at least four sets of four chambers (1,2,3,4), two sets of which are shown. Each is bounded by a series of alternate cation exchange membranes (5,7,9,11) and anion exchange membranes (6,8,10,12). Membranes 5 and 9 are dense types [CDS (Morgane)], membranes 6 and 10 are also dense types [ADS (Morgane)], membranes 7 and 11 are loose type [160% (Morgane)], and membranes 8 and 12 are also loose type [ADP (Morgane)].

Chamber 1 is connected via a conduit 15 to chamber 1 of an adjacent set, likewise chambers 2,3 and 4 are connected to chambers 2,3 and 4 of an adjacent set via conduits 16, 17 and 18 respectively. Each conduit 15, 16, 17, 18 is connected to a drain tap (not shown) so that liquid can be drained out of each chamber at the end of an electrodialysis cycle.

A mixture of amino acids to be separated, in the form of a protein hydrolysate, is introduced into chamber 3 in admixture with about 1 to 2% by weight of sodium chloride (as obtained from a previous demineralisation process), and sodium chloride solution is introduced into the remaining chambers.

Chamber 1 contains 0.5M sodium chloride, and each of chambers 2 and 4 contain 10⁻²M sodium chloride.

A voltage is applied across the ion exchange membranes and adjusted to obtain a current density of 30mA/cm². This causes acidic amino acids in chamber 3, together with chloride ions, to migrate across membrane 8 and accumulate in chamber 4. Further migration of negative ions through cation exchange membrane 9 is prevented since anion movement is blocked by the membrane 9. At the same time, basic amino acids, together with sodium ions, in chamber 3 migrate across membrane 7 and accumulate in chamber 2. Further migration of positive ions through anion exchange membrane 6 is prevented since cation movement is blocked by the membrane 6.

During the passage of current, sodium ions in each chamber 1 pass through cation membrane 5 or 9 into adjacent chamber 4, and chloride ions pass into chamber 2 through anion membranes 6 or 10, thereby depleting the concentration of sodium chloride in each chamber.

Eventually, all residual sodium chloride in chamber 3 will be removed and at that stage each chamber 1 is drained and an amount of water equal to the original volume of protein hydrolysate is added to the chamber. Current flow is then resumed and small quantities of 2M NaCl solution are added to each chamber 1 so the electrodialysis voltage does not exceed 6 volts. The current density is maintained at 5mA/cm². Also, small volumes of 5N sodium hydroxide are added continuously to each chamber 2 so that the pH does not exceed 9.5.

After further time has elapsed, all basic and amino acids in each chamber 3 will have been removed, leaving behind only the amphoteric acids. At that stage all chambers are drained off and the exchange membranes are subjected to a rinse cycle of about 20 minutes.

The rinses are performed with the following solutions, in the indicated sequence.

pure water; 0.1M HCl solution; 0.1M NaCl solution; 0.1M NaOH solution; 0.1M NaCl solution.

After the rinse cycle, further protein hydrolysate and salt solutions are introduced into the relevant chambers, and the electrodialysis process is repeated.

The protein hydrolysate employed in the electrodialysis process of the invention is derived from protein wastes, and before use must be decolourised and partially demineralised. Hydrolysates employed in the process include Keramine A (manufactured by the French company, Guyomarch) and Protein hydrolysate RW (manufactured by Diamalt) and these normally contain around 15 - 20% by weight of amino acids, and 13-15% by weight of sodium chloride. Decolourisation is carried out using active charcoal with a microfiltration system which allows regeneration and recovery of the charcoal.

Demineralisation is carried out using a conventional 2 chamber electrodialyser with compact, dense membranes such as Morgane membranes. The current density is fixed at 60ma/cm² and is maintained until the concentration of sodium chloride reaches 1 mol 1⁻¹. Then the current density is maintained at 30ma/cm² until the concentration of sodium chloride reaches 0.2 mol l⁻¹, and electrodialysis is stopped.

The amphoteric acid mixture drained from chamber 3 is preferably separated into its constituent acids using a standard 2 chamber electrodialyser.

Firstly, concentrated sodium hydroxide is added to the mixture in the first chamber in order to raise the pH to a value between 8.7 and 9.0. Salt solution in the second chamber is initially 10⁻²M.

With a current density of 30mA/cm², 60% to 80% of the glycine and serine components of the mixture is separated into the saline chamber. After emptying the saline chamber, further 10⁻²M sodium chloride is added and the electrodialysis continued until the movement of proline into the salt chamber becomes noticeable. Hence the first chamber becomes rich in proline and the saline chamber becomes rich in remaining amphoteric acids.

## Claims

1. A method of separating the acidic, basic and amphoteric components of an aqueous mixture of amino acids, which comprises introducing the mixture into a first electrodialyser chamber bounded by a cation exchange membrane and an anion exchange membrane, applying an electric potential across the chamber to cause the basic components to pass through the cation exchange membrane into a second electrodialyser chamber bounded by a further anion exchange membrane, and the acidic components to pass through the anion exchange membrane into a third electrodialyser chamber bounded by a further cationic exchange membrane, and maintaining the electric potential to permit the basic components and acidic components to accumulate in the second and third chambers respectively, thereby leaving the amphoteric components in the first chamber; characterised in that all chambers are subsequently drained and the various membranes are subjected to a rinse cycle in order to minimise fouling or clogging.

2. A method according to claim 1, wherein the rinse cycle comprises washing the membranes in a sequence consisting of water, dilute hydrochloric acid, dilute sodium chloride, dilute sodium hydroxide and finally dilute sodium chloride.

3. A method according to claim 1 or claim 2, wherein the amino acid mixture comprises a protein hydrolysate, basic amino acids and amphoteric acids.

4. A method according to any one of claims 1 to 3, wherein the second and third chambers contain an aqueous solution of a sodium salt as the conducting medium and a fourth electrodialysing chamber also containing a sodium salt is located adjacent to the second chamber, so as to create a set of four chambers in parallel.

5. A method according to any one of claims 1 to 4, wherein a plurality of sets of chambers are provided, wherein all the first chambers are connected to each other in series, and each second, third and optionally fourth chamber is also connected to the other second, third and optionally fourth chamber respectively.

6. A method according to claim 5, wherein at least four sets of chambers are provided.

7. A method according to any one of claims 1 to 6, wherein the cation and anion exchange membranes bounding the first chamber are of the 'loose' type, such as Morgane type CDS 160%, for the cation membrane, and Morgane type ADP, for the anion membrane.

8. A method according to any one of claims 1 to 7, wherein the anionic membrane bounding the second chamber, and the cationic membrane bounding the third chamber, are of the 'dense' type, such as Morgane type CDS, for the cation membrane, and Morgane type ADS for the anion membrane.

9. A method according to any one of claims 1 to 8, wherein an initial concentration of about 10⁻²M of salt in the second and third chambers is present and the initial concentration of salt in the fourth chamber is about 0.5M.

10. A method according to any one of claims 1 to 9, wherein the current density is from 20mAcm⁻² to 40mAcm⁻².

## Patentansprüche

1. Verfahren zur Trennung sauren basischen und amphoteren Komponenten eines wäßrigen Gemisches von Aminosäuren, umfassend Einführen des Gemisches in eine erste von einer Kationenaustauschermembran und einer Anionenaustauschermembran abgeschlossene Elektrodialysekammer, Anlegen eines elektrischen Potentials, das die basischen Komponenten dazu veranlaßt, durch die Kationenaustauschermembran in eine zweite von einer weiteren Anionenaustauschermembran abgeschlossene Elektrodialysekammer zu treten und die sauren Komponenten dazu veranlaßt, durch die Anionenaustauschermembran in eine dritte von einer weiteren Kationenaustauschermembran abgeschlossene Elektrodialysekammer zu treten und Halten des elektrischen Potentials, damit die basischen Komponenten und die sauren Komponenten sich in der zweiten bzw. dritten Kammer anreichern, wodurch die amphoteren Komponenten in der ersten Kammer zurückbleiben, dadurch gekennzeichnet, daß alle Kammern nacheinander entleert werden und die verschiedenen Membranen einem Spülzyklus unterzogen werden, damit Verschmutzen oder Verstopfen auf ein Minimum reduziert wird.

2. Verfahren nach Anspruch 1, wobei der Spülzyklus Waschen der Membranen nacheinander mit Wasser, verdünnter Salzsäure, verdünnter Natriumchloridlösung, verdünnter Natriumhydroxidlösung und schließlich verdünnter Natriumchloridlösung umfaßt.

3. Verfahren nach Anspruch 1 oder Anspruch 2, wobei das Aminosäuregemisch ein Proteinhydrolysat, basische Aminosäuren und amphotere Säuren umfaßt.

4. Verfahren nach einem der Ansprüche 1 bis 3, wobei die zweite und die dritte Kammer eine wäßrige Lösung eines Natriumsalzes als Leitmedium enthält und eine vierte Elektrodialysekammer, die ebenfalls Natriumsalz enthält, neben der zweiten Kammer so angeordnet ist, daß parallel ein Satz von vier Kammern entsteht.

5. Verfahren nach einem der Ansprüche 1 bis 4, wobei eine Vielzahl an Sätzen von Kammern bereitgestellt wird, alle ersten Kammern miteinander in Reihe geschaltet sind und jede zweite, dritte und gegebenenfalls vierte Kammer ebenfalls jeweils an die weitere zweite, dritte bzw. gegebenenfalls vierte Kammer angeschlossen ist.

6. Verfahren nach Anspruch 5, wobei mindestens vier Sätze von Kammern bereitgestellt werden.

7. Verfahren nach einem der Ansprüche 1 bis 6, wobei die Kationen- und die Anionenaustauschermembran, die die erste Kammer abschließen, vom "lockeren" Typ sind, wie CDS 160 %, Morgane für die Kationenmembran und ADP, Morgane, für die Anionenmembran.

8. Verfahren nach einem der Ansprüche 1 bis 7, wobei die anionische Membran, die die zweite Kammer abschließt und die kationische Membran, die die dritte Kammer abschließt, Membranen vom dichten Typ sind, wie CDS, Morgane, für die Kationenmembran und ADS, Morgane, für die Anionenmembran.

9. Verfahren nach einem der Ansprüche 1 bis 8, wobei eine anfängliche Konzentration von etwa 10⁻²M Salz in der zweiten und dritten Kammer vorliegt, und die anfängliche Konzentration an Salz in der vierten Kammer etwa 0,5 M ist.

10. Verfahren nach einem der Ansprüche 1 bis 9, wobei die Stromdichte 20 mAcm⁻² bis 40 mAcm⁻² beträgt.

## Revendications

1. Procédé pour séparer les constituants acides, basiques et amphotères d'un mélange aqueux d'aminoacides, qui comprend l'introduction du mélange dans une première chambre d'un appareil d'électrodialyse délimitée par une membrane échangeuse de cations et une membrane échangeuse d'anions, l'application d'un potentiel électrique à travers la chambre pour provoquer le passage du constituant basique à travers la membrane échangeuse de cations et à l'intérieur d'une deuxième chambre d'appareil d'électrodialyse délimitée par une autre membrane échangeuse d'anions, et le passage des constituants acides à travers la membrane échangeuse d'anions et à l'intérieur d'une troisième chambre d'appareil d'électrodialyse délimitée par une autre membrane échangeuse de cations, et le maintien du potentiel électrique pour permettre aux constituants basiques et aux constituants acides de s'accumuler respectivement dans les deuxième et troisième chambres, en laissant ainsi les constituants amphotères dans la première chambre ; caractérisé en ce que toutes les chambres sont ensuite vidées par écoulement et les différentes membranes sont soumises à un cycle de rinçage afin de réduire au minimum l'encrassement ou le colmatage.

2. Procédé suivant la revendication 1, dans lequel le cycle de rinçage comprend le lavage des membranes dans une séquence consistant en l'utilisation d'eau, d'acide chlorhydrique dilué, d'une solution de chlorure de sodium dilué, d'une solution d'hydroxyde de sodium dilué, et finalement d'une solution de chlorure de sodium dilué.

3. Procédé suivant la revendication 1 ou la revendication 2, dans lequel le mélange d'aminoacides comprend un hydrolysat de protéine, des aminoacides basiques et des acides amphotères.

4. Procédé suivant l'une quelconque des revendications 1 à 3, dans lequel les deuxième et troisième chambres contiennent une solution aqueuse d'un sel de sodium servant de milieu conducteur et une quatrième chambre d'électrodialyse contenant également un sel de sodium est située en position adjacente à la deuxième chambre, de manière à créer une série de quatre chambres parallèles.

5. Procédé suivant l'une quelconque des revendications 1 à 4, dans lequel une pluralité de séries de chambres est présente, dans laquelle toutes les premières chambres sont connectées les unes aux autres en série, chacune des deuxième et troisième et facultativement quatrième chambre est également connectée respectivement à l'autre des deuxième, troisième et facultativement quatrième chambres.

6. Procédé suivant la revendication 5, dans lequel au moins quatre séries de chambres sont présentes.

7. Procédé suivant l'une quelconque des revendications 1 à 6, dans lequel les membranes échangeuses de cations et d'anions délimitant la première chambre sont du type "lâche", tel qu'une membrane Morgane type CDS 160 % pour la membrane échangeuse de cations, et une membrane Morgane type ADP pour la membrane échangeuse d'anions.

8. Procédé suivant l'une quelconque des revendications 1 à 7, dans lequel la membrane anionique délimitant la deuxième chambre et la membrane cationique délimitant la troisième chambre sont du type "dense" tel qu'une membrane Morgane type CDS pour la membrane échangeuse de cations, et une membrane Morgane type ADS pour la membrane échangeuse d'anions.

9. Procédé suivant l'une quelconque des revendications 1 à 8, dans lequel une concentration initiale d'environ 10⁻²M de sel dans les deuxième et troisième chambres est présente et la concentration initiale de sel dans la quatrième chambre est égale à environ 0,5M.

10. Procédé suivant l'une quelconque des revendications 1 à 9, dans lequel la densité de courant est comprise dans l'intervalle de 20 mAcm⁻² à 40mAcm⁻².
